(19) Europäisches Patentamt
European Patent Office
Office européen des brevets

(11) Veröffentlichungsnummer: **0 005 186**
**B1**

(12) # EUROPÄISCHE PATENTSCHRIFT

(45) Veröffentlichungstag der Patentschrift: **30.12.81**

(51) Int. Cl.³: **C 07 D 261/08** // A61K31/42

(21) Anmeldenummer: **79101078.8**

(22) Anmeldetag: **09.04.79**

(54) **3-Alkyl-5-(2-hydroxy-styryl)-isoxazole und Verfahren zu ihrer Herstellung.**

(30) Priorität: **29.04.78 DE 2818998**

(43) Veröffentlichungstag der Anmeldung: **14.11.79 Patentblatt 79/23**

(45) Bekanntmachung des Hinweises auf die Patenterteilung: **30.12.81 Patentblatt 81/52**

(84) Benannte Vertragsstaaten: **BE CH DE FR GB NL**

(56) Entgegenhaltungen:
**DE-A-1 939 809**
**DE-A-2 549 962**

**HOUBEN-WEYL «Methoden der organischen Chemie», 4. Auflage, Band V/1d 1972, GEORG THIEME VERLAG, Stuttgart Seiten 127 bis 128**

(73) Patentinhaber: **BASF Aktiengesellschaft, Carl-Bosch-Strasse 38, D-6700 Ludwigshafen (DE)**

(72) Erfinder: **Thieme, Peter C., Dr., Dr.-Hans-Hoffmann-Strasse 5, D-6706 Wachenheim (DE)**
Erfinder: **Theobald, Hans, Dr., Parkstrasse 2, D-6703 Limburgerhof (DE)**
Erfinder: **Franke, Albrecht, Dr., Mandelring 11, D-6706 Wachenheim (DE)**
Erfinder: **Huber, Rolf, Dr., Pruemer Strasse 10, D-6700 Ludwigshafen 29 (DE)**

**EP 0 005 186 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 3-Alkyl-5-(2-hydroxy-styryl)-isoxazole und Verfahren zu ihrer Herstellung

Gegenstand der vorliegenden Erfindung sind 3-Alkyl-5-(2-hydroxy-styryl)-isoxazole der allgemeinen Formel (1)

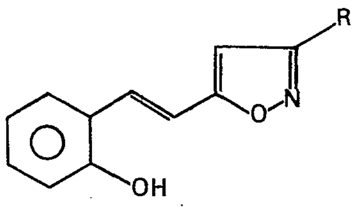

(1)

in der R einen Methyl-, Äthyl-, iso-Propyl- oder tert.-Butylrest bedeutet und Verfahren zu ihrer Herstellung.

Die erfindungsgemässen Verbindungen sind das 3-Methyl-5-(2-hydroxy-styryl)-isoxazol, 3-Äthyl-5-(2-hydroxy-styryl)-isoxazol, 3-iso-Propyl-5-(2-hydroxy-styryl)-isoxazol und 3-tert.-Butyl-5-(2-hydroxy-styryl)-isoxazol.

Die neuen Verbindungen der Formel (1) stellen wertvolle Zwischenprodukte für die Synthese von Arzneistoffen dar.

Die Verbindungen der Formel (1) können nach folgenden Verfahren hergestellt werden:

1. Durch Umsetzung eines Phosphonsäureesters der allgemeinen Formel (2)

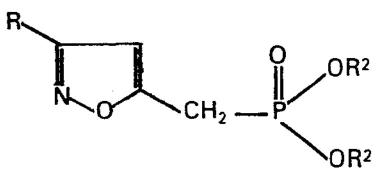

(2)

in der R die für Formel (1) angegebenen Bedeutungen hat und $R^2$ für einen niederen Alkylrest mit 1 bis 3 C-Atomen steht, mit einem substituierten Salicylaldehyd der allgemeinen Formel (3)

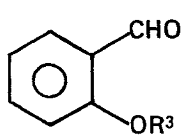

(3)

in der $R^3$ einen Alkoxyalkylrest der Formel (4)

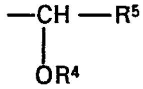

(4)

in der $R^4$ und $R^5$ gleich oder verschieden sind und einen niederen Alkylrest mit 1 bis 3 C-Atomen darstellen, wobei $R^5$ auch Wasserstoff und $R^3$ den Tetrahydropyranylrest bedeuten können, unter den an sich üblichen Bedingungen der Wittig-Horner-Reaktion zu einer Verbindung der allgemeinen Formel (5)

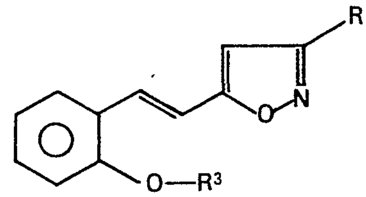

(5)

und nachfolgender Abspaltung der Schutzgruppe $R^3$ unter sauren Bedingungen.

Die Phosphonester der allgemeinen Formel (2) werden nach an sich bekannten Methoden aus den entsprechenden 3-Alkyl-5-chlormethyl-isoxazolen durch Umsetzung mit Trialkylphosphiten im Sinne einer Arbusov-Reaktion, wie es in Houben-Weyl, Bd. 12/1, S. 433 ff beschrieben ist, hergestellt. Die 3-Alkyl-5-chlormethyl-isoxazole lassen sich nach dem in der DOS 25 49 962 beschriebenen Verfahren herstellen. 3-Methyl-isoxazol-5-methylenphosphonat ist in der DOS 19 39 809 beschrieben und es wird dort seine Umsetzung mit 2-(Dimethylaminoäthoxy)-benzaldehyd zu 5-0-(2-Dimethylamino)äthoxy-styryl-3-methylisoxazol offenbart. Die in der vorliegenden Erfindung beanspruchten Verbindungen der Formel (1) und deren Herstellung werden in der DOS 19 39 809 nicht beschrieben und sind nach den dort offenbarten Verfahrensmassnahmen auch nicht nahegelegt.

Die Schutzgruppe $R^3$ stellt einen unter sauren Bedingungen leicht abspaltbaren, in alkalischem Milieu aber stabilen Rest dar, bevorzugt eine 1-Alkoxyalkylgruppe, wie den 1-Methoxyäthoxyrest. Salicylaldehyde der allgemeinen Formel (3) sind bekannt oder können nach bekannten Methoden herstestellt werden durch Umsetzung des Phenolates vom Salicylaldehyd mit 1-Chloralkylalkyl-äther, wie Chlormethyl-methyläther oder α-Chloräthyl-methyläther, oder durch Umsetzung von Salicylaldehyd mit Alkyl-vinyläthern unter saurer Katalyse. Die Synthese derartig geschützter Hydroxy-benzaldehyde ist beschrieben. Als Beispiele seien die folgenden Literaturstellen zitiert: P. Venturella, A. Bellino, Ann. Chimica 48, 706–722 (1958); R.L. Edwards, I. Mir, J. Chem. Soc. [London], Sect. C 1967, 411–13; DOS 22 09 128 und A. Arcoleo et al, Ann. Chimica 47, 667–74 (1957).

Die Umsetzung einer Verbindung der allgemeinen Formel (3) mit einem Phosphonester der allgemeinen Formel (2) erfolgt zweckmässigerweise in einem geeigneten Lösungsmittel in Gegenwart einer Base nach an sich üblichen Bedingungen, wie es beispielsweise in Houben-Weyl, Bd. 5/id, S. 127 ff und A. Maercker Org. Reactions, Bd. 14, S. 270 ff beschrieben ist. Als inerte Lösungsmittel kommen aromatische Kohlenwasserstoffe, wie Benzol, Toluol oder Xylol, höhersiedende aliphatische oder cycloaliphatische Äther, wie Äthylenglykol-dimethyläther, Tetrahydrofuran oder Dioxan, Dimethyl-formamid oder Dimethylsulfoxid oder Mischungen der genannten Lösungsmittel in Betracht. Die Umsetzungen werden vorteilhafterweise bei Raumtemperatur oder bei höheren Temperaturen von 30–80°C durchgeführt. Als Basen kommen Alkalimetallhydride, -amide oder -alkoholate, insbesondere die des Natriums und Kaliums, und Butyl- und Phenyllithium in Betracht.

Die Abspaltung der Schutzgruppe unter Freisetzung der erfindungsgemässen Verbindungen der Formel (1) aus den Verbindungen der Formel (5) wird nach an sich bekannten Methoden durchgeführt. In der Regel wird die erhaltene Verbindung der Formel (5) ohne vorherige Reinigung verseift. Die Abspaltung wird in saurem Milieu unter Verwendung einer anorganischen Säure, wie HCl oder $H_2SO_4$, oder mit Hilfe von sauren Ionenaustauscherharzen in einem geeigneten Lösungsmittel, wie Alkohole, Äther, Ketone, Kohlenwasserstoffe etc., insbesondere Methanol oder Äthanol, bei Raumtemperatur oder erhöhter Temperatur durchgeführt. Gegebenenfalls können auch Wasser/Alkohol-Gemische als Lösungsmittel verwendet werden. Bevorzugte Lösungsmittel sind Methanol/Wasser- und Aceton/Wasser-Gemische mit einer 1–10%igen Säurekonzentration.

2. Durch Umsetzung von Phosphoryliden der Formel (6)

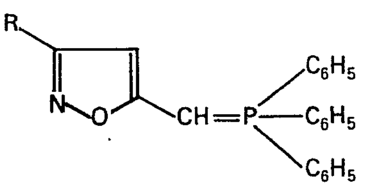

(6)

in der R die für Formel (1) angegebenen Bedeutungen hat, mit Salicylaldehyd oder einem geschützten Salicylaldehyd der allgemeinen Formel (3). Die Umsetzung erfolgt nach an sich bekannten Methoden, wie es beispielsweise in Houben-Weyl, Bd. 5/1b, S. 383 ff beschrieben wird. Bei der Verwendung von einem geschützten Salicylaldehyd wird die Schutzgruppe wie unter 1. beschrieben weiter abgespalten.

Bei den erfindungsgemässen Verfahren fallen die Verbindungen der allgemeinen Formel (1) als Gemische aus cis- und trans-Isomeren an, wobei in der Regel vorwiegend die trans-Verbindungen entstehen. Die cis- und die trans-Verbindungen lassen sich durch Kristallisation oder Säulenchromatographie ohne Schwierigkeiten voneinander abtrennen.

Die erfindungsgemässen Verbindungen der allgemeinen Formel (1) sind wertvolle Zwischenprodukte bei der Synthese pharmakologisch wirksamer Verbindungen. Beispielsweise können durch Alkylierung mit einem Epihalogenhydrin oder einem α,ω-Dihalogen-2-propanol und anschliessender Umsetzung in einem Amin Verbindungen der allgemeinen Formel (7)

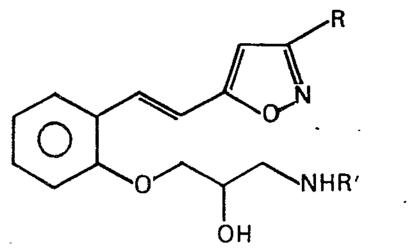

(7)

in denen R' insbesondere Isopropyl, tert.-Butyl, einen Cyclopropyl- oder einen 3-Methyl-1-butin-3-yl-rest bedeutet, erhalten werden, die zur Behandlung der Hypertonie, der coronaren Herzkrankheit und von Herzrhythmusstörungen verwendet werden können.

Die folgenden Beispiele sollen die Erfindung näher erläutern.

Herstellung der Ausgangsmaterialien

Beispiel I: o-(α-Methoxy-äthoxy)-benzaldehyd

a) 610 g (5 Mol) Salicylaldehyd werden in 1,5 l Xylol gelöst; zu dieser Lösung werden bei 40–50°C 900 g (5 Mol) einer 30%igen $NaOCH_3$-Lösung in Methanol zugegetropft. Anschliessend wird aufgeheizt und das Methanol abdestilliert, welches im Reaktionskolben nach und nach durch die gleiche Menge Xylol ersetzt wird. Es wird so lange erhitzt, bis das Xylol abzudestillieren beginnt (ca. 130°C am Destillationsübergang). Dann wird die Suspension des Na-Salzes des Salicylaldehyds auf 60°C abgekühlt und wie unter c) beschrieben weiter umgesetzt.

b) 200 ml Xylol werden mit einer Spatelspitze Hydrochinon versetzt und auf −20 bis −30°C abgekühlt und 290 g (5 Mol) Vinyl-methyläther einkondensiert. Anschliessend werden bei −30°C 183 g (5 Mol) HCl eingegast und die Lösung durch Stehenlassen auf Raumtemperatur erwärmt. Die Lösung des erhaltenen 1-Chloräthyl-methyläthers wird wie unter c) beschrieben weiter umgesetzt.

c) Die wie unter b) beschrieben hergestellte Lösung des 1-Chloräthylmethyläthers wird zu der 60°C heissen Lösung des Na-Salzes des Salicylaldehyds (siehe a) zugetropft und noch ca. 1½ Stunden bei 60°C weitergerührt, gegebenenfalls wird mit Hilfe von 30%iger $NaOCH_3$-Lösung ein pH-Wert von 8–9 eingestellt und über Nacht bei Raumtemperatur weitergerührt.

Anschliessend wird vom ausgefallenen Natriumchlorid abfiltriert, nachgewaschen mit Xylol und am Rotationsverdampfer das Xylol abdestilliert. Der verbleibende Rückstand wird bei 2 mm Hg über eine Kolonne destilliert. Es werden 690 g o-(α-Methoxyäthoxy)-benzaldehyd vom $Kp_2$: 94–96°C gewonnen.

Beispiel II: (3-Methylisoxazolyl-5)-methanphosphonsäurediäthylester

445 g 5-Chlormethyl-3-methylisoxazol und 674 g Phosphorigsäuretriäthylester werden langsam auf 150°C erhitzt und 4 Stunden bei dieser Temperatur belassen. Nach der Destillation erhält man 546 g (69% d. Theorie) (3-Methylisoxazolyl-5)-methanphosphonsäurediäthylester vom Sdp. 118–121°C/0,3 Torr.

$^1$H-NMR-Spektrum ($CHCl_3$, TMS intern):
τ = 3,85 (d, J = 3 Hz, 1 H), 4,17 (m, J = 8 Hz, 4H), 6,67 (d, J = 22 Hz, 2H), 7,72 (s, 3H), 8,67 (t, J = 8 Hz, 6H)

$C_9H_{16}NO_4P$ (233,21)

| | | | | |
|---|---|---|---|---|
| Ber.: | C 46,35% | H 6,91% | N 6,01% | P 13,28% |
| Gef.: | C 45,9 % | H 7,0 % | N 6,0 % | P 13,0 % |

Beispiel III: (3-Äthylisoxazolyl-5)-methanphosphonsäurediäthylester

15 g 5-Chlormethyl-3-äthyl-isoxazol und 18 g Triäthylphosphit werden langsam auf 150°C aufgeheizt und 2½ Stunden bei dieser Temperatur belassen. Nach dem Abkühlen wird der Rückstand unter vermindertem Druck destilliert. Man erhält 18,2 g (3-Äthylisoxazolyl-5)-methanphosphonsäurediäthylester vom Sdp. 120–121°C/0,2 mmHg (Ausbeute: 71,2%)

$^1$H-NMR-Spektrum ($CDCl_3$, TMS intern):
τ = 3,85 (d, J = 3 Hz, 1H), 4,17 (m, J = Hz, 4H), 6,60 (d, H = 20 Hz, 2H), 7,35 (q, J = Hz, 2H), 8.50 - 8,93 (m, 9H)

$C_{10}H_{18}NO_4P$ (247,23)

| | | | | |
|---|---|---|---|---|
| Ber.: | C 48,58% | H 7,34% | N 5,67% | P 12,53% |
| Gef.: | C 48,4 % | H 7,1 % | N 5,7 % | P 12,3 % |

Die weiteren Phosphonester werden analog hergestellt
(3-Isopropyl-isoxazolyl-5)-methanphosphonsäurediäthylester

$Kp_{0,3}$: 117–122°C (Ausbeute 73%)

(3-tert.-Butyl-isoxazolyl-5)-methanphosphonsäurediäthylester

$Kp_{0,3}$: 126–132°C (Ausbeute 88%)

Herstellung
von erfindungsgemässen Verbindungen

Beispiel 1
3-Methyl-5-(2-hydroxy-styryl)-isoxazol
8,8 g (0,2 Mol) 55% Natriumhydridsuspension in Paraffinöl werden in 100 ml absolutem Dimethylsulfoxid vorgelegt. Dazu werden 47 g (0,2 Mol) Diäthyl-(3-methyl-isoxazolyl-5)-methylenphosphonat bei Raumtemperatur getropft. Man lässt 30 Minuten nachrühren und tropft anschliessend unter Rühren 36 g (0,2 Mol) o-(1-Methoxy-äthoxy)-benzaldehyd zu und lässt das Reaktionsgemisch für 24 Stunden bei Raumgemperatur rühren. Man giesst auf 1 l Eiswasser und extrahiert 3 mal mit je 80 ml Methylenchlorid. Die vereinigten organischen Phasen werden mit Natriumsulfat getrocknet und am Rotationsverdampfer eingeengt. Der ölige Rückstand wird in 80 ml Methanol und 10 ml Wasser gelöst und mit 2 ml 5 n HCl versetzt und für 10 Minuten nachgerührt. Dann wird langsam ein Überschuss an Wasser zum Gemisch gegeben, bis ein Niederschlag ausfällt. Dieser wird abgesaugt, mit Wasser gewaschen und aus Äthanol umkristallisiert. 19 g (47% d. Th.) farblose Kristalle, Fp. 236–238°C.

$C_{12}H_{11}NO_2$ (201)

| | | | |
|---|---|---|---|
| Ber.: | C 71,6 | H 5,5 | N 7,0 |
| Gef.: | C 71,8 | H 5,5 | N 6,8 |

Beispiel 2
3-Äthyl-5-(2-hydroxy-styryl)-isoxazol
Analog Beispiel 1 werden 6 g (0,02 Mol) Diäthyl-(3-äthyl-isoxazolyl-5)-methylenphosphonat und 4,4 g (0,02 Mol) o-(1-Methoxy-äthoxy)-benzaldehyd umgesetzt und aus Isopropanol umkristallisiert, 7 g (32% d. Th.) farblose Kristalle.

Fp. 175–176 °C

$C_{13}H_{13}NO_2$ (215)

| | | | |
|---|---|---|---|
| Ber.: | C 72,5 | H 6,1 | N 6,5 |
| Gef.: | C 72,5 | H 6,2 | N 6,6 |

Beispiel 3
3-Isopropyl-5-(2-hydroxy-styryl)-isoxazol
Analog Beispiel 1 werden 32 g (0,12 Mol) Diäthyl-(3-isopropyl-isoxazolyl-5)-methylenphosphonat und 22 g (0,12 Mol) o-(1-Methoxy-äthoxy)-benzaldehyd umgesetzt und aus Toluol umkristallisiert. 20 g (73% d. Th.) farblose Kristalle, Fp. 129–133°C.

$C_{16}H_{15}NO_2$ (229)

| | | | |
|---|---|---|---|
| Ber.: | C 73,3 | H 6,6 | N 6,1 |
| Gef.: | C 73,7 | H 6,7 | N 5,8 |

Beispiel 4
3-tert.-Butyl-5-(2-hydroxy-styryl)-isoxazol
Analog Beispiel 1 werden 35 g (0,13 Mol) Diäthyl-(3-tert.-butyl-isoxazolyl-5)-methylenphosphonat und 23 g (0,13 Mol) o-(1-Methoxy-äthoxy)-benzaldehyd umgesetzt und aus Toluol umkristallisiert.

24,8 g (78% d. Th.) farblose Kristalle, Fp. 152–155 °C

$C_{15}H_{17}NO_2$ (243)

Ber.: C 74,0 H 7,0 N 5,8
Gef.: C 73,4 H 7,3 N 5,5

**Patentansprüche**

1. 3-Methyl-5-(2-hydroxy-styryl)-isoxazol.
2. 3-Äthyl-5-(2-hydroxy-styryl)-isoxazol.
3. 3-iso-Propyl-5-(2-hydroxy-styryl)-isoxazol.
4. 3-tert.-Butyl-5-(hydroxy-styryl)-isoxazol.
5. Verfahren zur Herstellung von 3-Alkyl-5-(2-hydroxy-styryl)-isoxazolen der allgemeinen Formel (1)

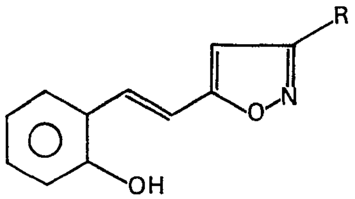

(1)

in der R einen Methyl., Äthyl., iso-Propyl. oder test. Butylrest bedeutet, dadurch gekennzeichnet, dass man einen Phosphonsäureester der allgemeinen Formel (2)

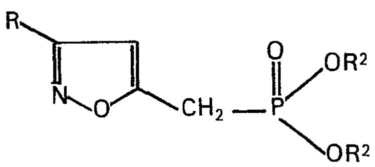

(2)

in der R die für Formel (1) angegebenen Bedeutungen hat und $R^2$ für einen niederen Alkylrest mit 1 bis 3 C-Atomen steht, mit einem substituierten Salicylaldehyd der allgemeinen Formel (3)

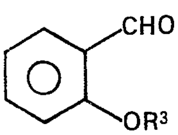

(3)

in der $R^3$ einen Alkoxyalkylrest der Formel (4)

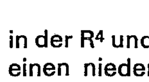

(4)

in der $R^4$ und $R^5$ gleich oder verschieden sind und einen niederen Alkylrest mit 1 bis 3 C-Atomen darstellen, wobei $R^5$ auch Wasserstoff und $R^3$ den Tetrahydropyranylrest bedeuten können, unter an sich üblichen Bedingungen umsetzt udn anschliessend die Schutzgruppe $R^3$ unter sauren Bedingungen abspaltet.

**Claims**

1. 3-Methyl-5-(2-hydroxy-styryl)-isoxazole.
2. 3-Ethyl-5-(2-hydroxy-styryl)-isoxazole.
3. 3-iso-Propyl-5-(2-hydroxy-styryl)-isoxazole.
4. 3-tert.-Butyl-5-(hydroxy-styryl)-isoxazole.
5. A process for the preparation of a 3-alkyl-5-(2-hydroxy-styryl)-isoxazole of the general formula (1)

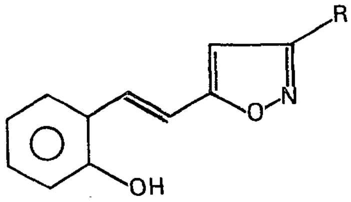

(1)

where R is methyl, ethyl, isopropyl or tert.-butyl, characterized in that a phosphonic acid ester of the general formula (2)

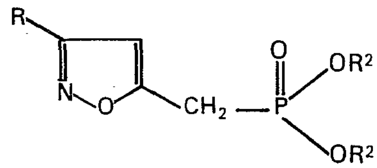

(2)

where R has the meanings given for formula (1) and $R^2$ is lower alkyl of 1 to 3 carbon atoms, is reacted with a substituted salicylaldehyde of the general formula (3)

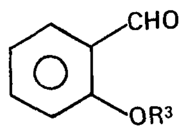

(3)

where $R^3$ is alkoxyalkyl of the formula (4)

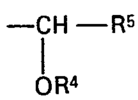

(4)

(where $R^4$ and $R^5$ are identical or different and each is lower alkyl of 1 to 3 carbon atoms, and $R^5$ can also be hydrogen) or $R^3$ is tetrahydropyranyl, under conventional conditions, after which the protective group $R^3$ is detached under acid conditions.

**Revendications**

1. 3-méthyl-5-(2-hydroxy-styryl)-isoxazole.
2. 3-éthyl-5-(2-hydroxy-styryl)-isoxazole.
3. 3-iso-propyl-5-(2-hydroxy-styryl)-isoxazole.
4. 3-tert.-butyl-5-(hydroxy-styryl)-isoxazole.

5. Procédé de préparation de 3-alkyl-5-(2-hydroxy-styryl)-isoxazoles de formule générale (1)

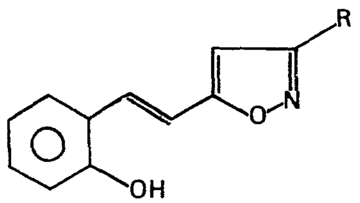

(1)

dans laquelle R représente un reste méthyle, éthyle, isopropyle ou tert.-butyle, caractérisé par le fait qu'on fait réagir, dans des conditions habituelles, un ester d'acide phosphonique de formule générale (2)

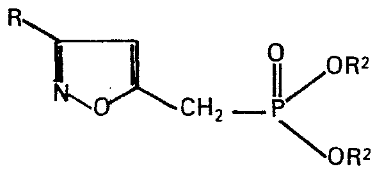

(2)

dans laquelle R a la signification indiquée pour la formule (1) et $R^2$ représente un reste alkyle inférieur à 1 à 3 atomes C, avec un aldéhyde salicylique substitué de formule générale (3)

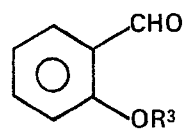

(3)

dans laquelle $R^3$ représente un reste alkoxyalkyle de formule (4)

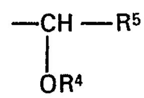

(4)

dans laquelle $R^4$ et $R^5$ sont identiques ou différents et représentent un reste alkyle inférieur à 1 à 3 atomes C, $R^5$ pouvant représenter ainsi l'hydrogène et $R^3$ le reste tétrahydropyranyl et l'on sépare ensuite les groupes protecteurs $R^3$ dans des conditions acides.